**Europäisches Patentamt**

**European Patent Office**

**Office européen des brevets**

(19)

(11) Veröffentlichungsnummer: **0 004 680**
**B1**

(12) # EUROPÄISCHE PATENTSCHRIFT

(45) Veröffentlichungstag der Patentschrift:
14.10.81

(21) Anmeldenummer: 79101114.1

(22) Anmeldetag: 11.04.79

(51) Int. Cl.³: **C 07 C 175/00**, A 61 K 31/20,
A 61 K 31/23

(54) **Nonatetraensäure-Derivat und pharmazeutische Kompositionen.**

(30) Priorität: 12.04.78 US 895942

(43) Veröffentlichungstag der Anmeldung:
17.10.79 Patentblatt 79/21

(45) Bekanntmachung des Hinweises auf die Patenterteilung:
14.10.81 Patentblatt 81/41

(84) Benannte Vertragsstaaten:
CH DE FR GB IT

(56) Entgegenhaltungen:
1. **CHEMICAL ABSTRACTS, vol. 77, Nr. 25, 18. Dezember 1972, Seite 435, Nr. 164875e, Columbus, Ohio USA
RAO, M. S. SUREKHA et al., »Vitamin A2. Preparation, properties, metabolism and biological activity of 4-oxoretinaic acid«
2. TETRAHEDRON LETTERS, Nr. 18 (1972), Seiten 1823—25 Pergamon Press, Osford, GB A. B. BARUA et al., »Preparation and properties of 4-oxo-retinaic acid and its methylester«**

(73) Patentinhaber: **F. HOFFMANN-LA ROCHE & CO.
Aktiengesellschaft, CH-4002 Basel (CH)**

(72) Erfinder: **Hänni, Ralph, Dr., Mittlerer Rainweg,
CH-4414 Füllinsdorf (CH)**
Erfinder: **Ryser, Gottlieb, Lange Gasse 1, CH-4052 Basel
(CH)**

(74) Vertreter: **Lederer, Franz, Dr. et al, Patentanwälte Dr.
Lederer Franz Meyer-Roxlau Reiner F.
Lucile-Grahn-Strasse 22, D-8000 München 80 (DE)**

## Nonatetraensäure-Derivat und pharmazeutische Kompositionen

Erfindungsgemäß wurde gefunden, daß die Verbindung 9-(2,6,6-Trimethyl-3-hydroxy-1-cyclohexenyl)-3,7-dimethyl-2,4,6,8-nonatetraensäure und pharmazeutisch anwendbare Salze davon als Mittel gegen Tumoren von Wert sind.

Die vorliegende Erfindung betrifft dementsprechend die erwähnte Verbindung sowie neue pharmazeutische Kompositionen, die diese Verbindung oder ein pharmazeutisch anwendbares Salz davon enthalten. Die neue Verbindung bzw. die neuen Kompositionen können zur Prophylaxe gewisser prämaligner und karzinomatöser Zustände durch systemische Verabreichung und zur therapeutischen Behandlung von Karzinomen, insbesondere solchen der Haut, des Gastrointestinaltraktes, des Respirationstraktes, des Urogenitaltraktes, der Milchdrüsen und der Blase durch systemische Verabreichung Verwendung finden.

Die 9-(2,6,6-Trimethyl-3-oxo-1-cyclohexenyl)-3,7-dimethyl-2,4,6,8-nonatetraensäure und ihr Methylester sind aus Chemical Abstracts Vol. 77 (1972), 16 4875 e bekannt. Aus dieser Publikation ist ferner bekannt, daß die genannten Verbindungen bei Ratten eine geringere Vitamin-A-Aktivität besitzen als Vitamin-A-Säure.

Aus Tetrahedron Letters No. 18 (1972), S. 1823—1825 ist der 9-(2,6,6-Trimethyl-3-hydroxy-1-cyclohexenyl)-3,7-dimethyl-2,5,6,8-nonatetraensäuremethylester bekannt, ohne daß in dieser Publikation Angaben über eine biologische Wirkung dieses Esters gemacht werden.

Die neue 9-(2,6,6-Trimethyl-3-hydroxy-1-cyclohexenyl)-3,7-dimethyl-2,4,6,8-nonatetraensäure kann aus der entsprechenden, bekannten 3-oxo-Verbindung durch Reduktion mit einem Alkalimetallborhydrid in an sich bekannter Weise hergestellt werden.

Die Verbindung 9-(2,6,6-Trimethyl-3-hydroxy-1-cyclohexenyl)-3,7-dimethyl-2,4,6,8-nonatetraensäure zeigt eine reduzierende Wirkung auf das Wachstum von Tumoren wie Papillomen und Chondrosarcomen. Bei der Verabreichung an Ratten mit transplantierten Chondrosarcomen wurde eine wesentliche Hemmung des Wachstums der Tumoren beobachtet.

Die neue Verbindung zeigte dabei bei einer Verabreichung von 20 mg/kg/Tag intraperitoneal während der Dauer von 4 Wochen 94% bzw. 95% Hemmung ohne jede Toxizität. Die Hemmung wurde durch Vergleich des Gewichtes von Tumoren von behandelten Ratten mit solchen unbehandelten Ratten gemessen.

Die neue Verbindung ist ebenfalls von Wert als Medikament für die topische und systemische Therapie von Akne, Psoriasis und verwandten dermatologischen Symptomen, die durch eine vermehrte oder pathologisch veränderte Verhornung gekennzeichnet sind, und ebenso bei inflammatorischen und allergischen dermatologischen Zuständen. Sie kann weiterhin Verwendung finden, um Krankheiten zu behandeln, die durch inflammatorische oder degenerative Veränderung der Schleimhäute gekennzeichnet sind.

Die neue Verbindung kann infolgedessen als Medikament, beispielsweise als pharmazeutisches Präparat verwendet werden, welches sie zusammen mit einem verträglichen pharmazeutischen Träger enthält. Pharmazeutische Präparate für systemische Verabreichung können z. B. durch Vermischen der neuen Verbindung als wirksamen Inhaltsstoff mit in solchen Präparaten üblichen, nicht toxischen, inerten, festen oder flüssigen Trägern hergestellt werden. Die pharmazeutischen Präparate können enteral oder parenteral verabreicht werden. Geeignete pharmazeutische Präparate für enterale Verabreichung sind beispielsweise Tabletten, Kapseln, Dragées, Sirupe, Suspensionen, Lösungen und Supositorien. Geeignete parenterale Verabreichungsformen sind Infusions- oder Injektionslösungen.

Die pharmazeutischen Präparate können inerte oder andere pharmacodynamisch wirksame Zusätze enthalten. Tabletten oder Granulate können beispielsweise Bindemittel, Füllstoffe, Trägermaterialien oder Verdünnungsmittel enthalten. Flüssige Präparate können beispielsweise als sterile wassermischbare Lösung vorliegen. Kapseln können zusätzlich einen Füllstoff oder Verdickungsmittel enthalten. Weiterhin können geschmacksverbessernde Zusatzstoffe und Substanzen verwendet werden, die üblicherweise als Konservierungsmittel, Stabilisierungsmittel, Feuchthaltemittel oder Emulgatoren angewendet werden, weiterhin können Salze zur Veränderung des osmotischen Druckes, Puffer und andere Zusatzstoffe anwesend sein.

Die erwähnten Trägerstoffe und Verdünnungsmittel können organische oder anorganische Substanzen sein, beispielsweise Wasser, Gelatine, Laktose, Stärke, Magnesiumstearat, Talk, Gummi arabicum, Polyalkylenglykole. Selbstverständlich müssen alle bei der Herstellung der pharmazeutischen Kompositionen verwendeten Hilfsstoffe nicht-toxisch sein. Für topische Anwendung wird die neue Verbindung zweckmäßig in die Form von Salben, Tinkturen, Crèmes, Lösungen, Lotionen, Sprays oder Suspensionen gebracht. Salben und Crèmes sowie Lösungen sind bevorzugt. Diese pharmazeutischen Kompositionen für topische Verabreichung können durch Mischen der Polyenverbindungen als Wirkstoff mit nicht-toxischen, inerten, festen oder flüssigen Trägern, die an sich für die Anwendung für topische Präparate bekannt sind, hergestellt werden. Besonders bevorzugt für die topische Verabreichung sind ca. 0,01%ige bis ca. 0,3%ige (insbesondere 0,02%ige bis 0,1%ige) Lösungen und ca. 0,05%ige bis ca. 5%ige (insbesondere 0,1%ige bis ca. 2%ige Salben oder Crèmes). Gewünschtenfalls kann ein Antioxidans (z. B. Tocopherol, N-Methyl-$\gamma$-tocopheramin, butyliertes

2

Hydroxyanisol oder butyliertes Hydroxytoluol) der pharmazeutischen Komposition zugesetzt werden.

Bevorzugte pharmazeutisch anwendbare Salze der neuen Verbindung sind die Ammoniumsalze, Alkalisalze und Erdalkalimetallsalze. Besonders bevorzugt ist das Natriumsalz, das Calciumsalz, das Ammoniumsalz und substituierte Ammoniumsalze wie Mono-, Di- oder Trialkylammoniumsalze.

Die neue Verbindung bzw. ihre Salze kann systematisch auch Patienten verabreicht werden, die für Karzinome insbesondere solche der oben erwähnten Art, empfänglich sind. Gewisse Karzinome, die erfindungsgemäß verhütet werden sollen, verlaufen häufig durch ein bestimmtes prämalignes Stadium, das als solches diagnostiziert werden kann. Statistisch gesehen entwickeln sich 20—30% solcher prämalignen Zustände in Karzinome. Jeder einzelne Fall hat indessen eine Neigung, sich zum Karzinom zu entwickeln, wenn er unbehandelt bleibt. In solchen Fällen kann die systematische Behandlung eines prämalignen Zustandes dazu dienen, die Entwicklung eines Karzinoms zu verhüten. In anderen Fällen kann ein Individium, ohne daß ein prämaligner Zustand positiv diagnostiziert werden kann, für ein Karzinom empfänglich sein, so daß eine Behandlung mit einer erfindungsgemäßen pharmazeutischen Komposition angezeigt ist.

Die Terminologie »Karzinom«, die hier im Zusammenhang mit der erfindungsgemäßen prophylaktischen Verwendung gebraucht wird, umfaßt Karzinome, welche die Epithelien diverser Körperteile betreffen, z. B. geschichtetes Plattenepithel von Haut und Schleimhäuten, Zylinderepithel des Intestinaltraktes, das Epithel des Urogenitaltraktes und Cilien-tragende Epithelien von Teilen des Respirationstraktes wie z. B. der oberen Atemwege bzw. pseudogeschichtete Epithelien von Trachea und Bronchien. Karzinome, welche diese Zellen befallen, schließen diejenigen des Epithels der Haut, Zunge, Harnblase, Brustdrüse, Pharynx, Kehlkopf, Bronchien, Oesophagus, Magen, Dickdarm, Gebärmutter und Vulva ein.

Die tägliche Dosierung der neuen Verbindung hängt von den Bedürfnissen des Patienten ab, insbesondere in den Fällen, wo ein bestimmter prämaligner Zustand diagnostiziert worden ist. Im allgemeinen beträgt die tägliche Dosierung bei enteraler oder parenteraler Administration etwa 0,05 mg bis etwa 3 mg pro kg Körpergewicht. Besonders bevorzugt ist eine Dosierung von etwa 0,1 mg bis etwa 1 mg pro kg. Die Dosierung kann nach den Vorschriften des Arztes im Hinblick auf die Bedürfnisse des Patienten, der Existenz des prämalignen Zustandes und anderer Faktoren, wie des Alters des Patienten, nach einem geeigneten Schema verabreicht werden.

Der nachstehend beschriebene Test zeigt, daß die neue Verbindung ein wirksames Antitumormittel darstellt, das bei weiblichen Ratten das Wachstum von Chondrosarkomen hemmt. Bei diesem Test werden mehrere kleine Stücke des Chondrosarkoms subcutan mittels eines Trocars in die rechte Leistenregion von 40—50 g schweren Ratten implantiert. Nach 30—40 Tagen werden Gruppen von 8 Tieren gebildet und man beginnt mit der intraperonealen Behandlung, welche fünfmal pro Woche erfolgt. Die Behandlung wird 4 Wochen fortgesetzt, danach werden die Tiere getötet, die Tumoren herausgeschnitten und gewogen.

Die Hemmung des Tumorwachstums wird nach der Formel

$$\frac{C-T}{C} \cdot 100\%$$

berechnet, wobei C und T das mittlere Tumorgewicht der unbehandelten Kontrolltiere (C) und der behandelten Tiere (T) darstellen. Die Resultate sind als % der Hemmung des Tumorwachstums angegeben. Wenn die beobachtete Hemmung 50% oder größer ist, wird die Aktivität als wirksam beurteilt. Wenn die Hemmung geringer als 50% ist, wird dies als inaktiv bezeichnet. Die Resultate sind in der nachstehenden Tabelle zusammengefaßt:

Wachstumshemmung bei Chondrosarcomen

| Dosierung | Hemmung | Änderung des Körpergewichts | |
|---|---|---|---|
| mg/kg/Tag | % | Behandelt | Kontrollen |
| 20 | 94 | +30 | +34 |
| 10 | 86 | +22 | +29 |
| 5 | 41 | +31 | +29 |

Die Toxizität der neuen Verbindung wird durch Syndrome der toxischen A-Hypervitaminose bestimmt. Die neue Verbindung ist verhältnismäßig untoxisch, gemessen an diesem Syndrom, was sie zur internen Verabreichung, z. B. peroral, intravenös oder intraperitoneal geeignet macht.

Das Syndrom der toxischen A-Hypervitaminose wird nach einer in J. Cancer, Vol. 10 (1974) Seite 731—733 erläuterten Methode berechnet. Der Vitamin-A-Hypervitaminose-Test wird bei Mäusen im Gewicht von 25—27 g ausgeführt, die 14 Tage lang 10 intraperitoneale Injektionen der Testsubstanz, suspendiert in Arachisöl, erhalten. Man erhält die folgenden Symptome, die in einem Bereich von 0−4 ausgewertet werden:

| | Stufe | | | | |
|---|---|---|---|---|---|
| | 0 | 1 | 2 | 3 | 4 |
| Gewichtsverlust | <1 g | 1−3 g | 4−6 g | 7−9 g | ⩾10 g |
| Hautschuppung | keine | schwach | mäßig | stark | sehr stark |
| Haarausfall | keine | schwach | mäßig | stark | sehr stark |
| Knochenbrüche der Extremitäten (Anzahl, makroskopische Beobachtung) | 0 | 1 | 2 | 3 | >4 |

Die A-Hypervitaminose wird als der Zustand definiert, bei dem die Addition aller Symptomstufen mindestens einen Wert von 3 ergibt. Man bestimmt die niedrigste tägliche Dosis, die bei dieser 14-Tage-Studie in der Lage ist, eine A-Hypervitaminose zu verursachen. Die neue Verbindung erzeugte bei dieser Studie eine A-Hypervitaminose bei einer Dosierung von über 200 mg pro kg pro Tag.

Die tumorhemmende Aktivität der neuen Verbindung wurde weiter durch systemische Behandlung von Papillomen, die nach der Dimethyl-benzanthracen-Crotonöl-Methoden induziert worden waren, nachgewiesen. Bei dieser Methode werden Albinomäuse zweimal täglich in Abständen von 15 Tagen auf einer 5 cm²-Region des Rückens mit 150 $\gamma$ Dimethylbenzanthracen (DMBA) gelöst in 0,2 ml Aceton gepinselt. 3 Wochen nach der zweiten Pinselung mit DMBA wurden die Gebiete mit 0,5 mg Crotonöl, gelöst in 0,2 mg Aceton, zweimal wöchentlich im Verlauf von 3−4 Monaten bis zum Auftreten von Papillomen gepinselt. Sobald die Papillome, meistens multiple Papillome, einen Durchmesser von etwa 4 mm erreicht hatten, wurde mit der therapeutischen Behandlung begonnen. Gruppen von 4 Mäusen erhielten die Testverbindung einmal wöchentlich während 4 Wochen. Die Verabreichung wurde bei verschiedenen Gruppen, sowohl p. o. als auch i. p. vorgenommen. Am ersten und fünfzehnten Therapietage wurde der mittlere Radius der Papillome gemessen. Die Änderung des mittleren Papillomvolumens von Tag 1 bis zum Tag 15 wurde bestimmt und als Prozentänderung des Volumens am Tage 1 ausgedrückt. Eine Kontrollgruppe erhielt keine Medikation. Die Resultate dieses Testes sind nachstehend angegeben.

| Dosis | Regression |
|---|---|
| 200 mg/kg pro Woche | 34% |
| 100 mg/kg pro Woche | 28% |

Die folgenden Beispiele illustrieren die Erfindung weiter:

### Beispiel 1

Eine Lösung von 1,5 g (E,E,E,E)-9-(2,6,6-Trimethyl-3-oxo-1-cyclohexenyl)-3,7-dimethyl-2,4,6,8-nonatetraensäure in 25 ml Äthanol wurde auf 5° gekühlt, mit 200 mg Natriumborhydrid versetzt und 30 Minuten bei Raumtemperatur gerührt. Danach wurde Essigsäure zugegeben und die Lösungsmittel unter vermindertem Druck abgedampft. Der Rückstand wurde mit Dichlormethan extrahiert und lieferte nach Eindampfen und Kristallisation aus Äthylacetat reine (E,E,E,E)-9-(2,6,6-Trimethyl-3-hydroxy-1-cyclohexenyl)-3,7-dimethyl-2,4,6,8-nonatetraensäure vom Schmelzpunkt 181−183°.

## 0 004 680

### Beispiel 2

Dragées wurden in an sich bekannter Weise aus den folgenden Inhaltsstoffen hergestellt:

|  | Menge/Dragée |
|---|---|
| (E,E,E,E)-9-(2,6,6-Trimethyl-3-hydroxy-1-cyclohexenyl)-3,7-dimethyl-2,4,6,8-nonatetraensäure | 16,0 mg |
| Lactose | 40,0 mg |
| Maisstärke | 40,0 mg |
| Talk | 3,5 mg |
| Stearinsäure | 0,5 mg |
| Überzug | 120,0 mg |
| Gesamtgewicht | 220,0 mg |

### Beispiel 3

Hartgelatinekapseln wurden mit folgender Komposition gefüllt:

|  | Menge/Kapsel |
|---|---|
| (E,E,E,E)-9(2,6,6-Trimethyl-3-hydroxy-1-cyclohexenyl)-3,7-dimethyl-2,4,6,8-nonatetraensäure | 20,0 mg |
| Talk | 8,0 mg |
| Lactose | 152,0 mg |
| Gesamtgewicht | 180,0 mg |

### Beispiel 4

Weichgelatinekapseln wurden mit folgender Komposition gefüllt:

|  | Menge/Kapsel |
|---|---|
| (E,E,E,E)-9(2,6,6-Trimethyl-3-hydroxy-1-cyclohexenyl)-3,7-dimethyl-2,4,6,8-nonatetraensäure | 20,0 mg |
| Wachsmischung | 51,5 mg |
| Vegetabiles Öl | 103,0 mg |
| Sequestrin[1]) | 0,5 mg |
| Gesamtgewicht | 175,0 mg |

[1]) Natriumsalz der Äthylendiamintetraessigsäure.

5

**0 004 680**

**Patentansprüche**

1. 9-(2,6,6-Trimethyl-3-hydroxy-1-cyclohexenyl)-3,7-dimethyl-2,4,6,8-nonatetraensäure oder ein pharmazeutisch anwendbares Salz davon.

2. Pharmazeutische Komposition enthaltend die Verbindung 9(2,6,6-Trimethyl-3-hydroxy-1-cyclohexenyl)-3,7-dimethyl-2,4,6,8-nonatetraensäure oder ein pharmazeutisch anwendbares Salz davon.

**Claims**

1. 9-(2,6,6-Trimethyl-3-hydroxy-1-cyclohexenyl)-3,7-dimethyl-2,4,6,8-nonatetraenoic acid or a pharmaceutically usable salt thereof.

2. Pharmaceutical composition containing the compound 9-(2,6,6-trimethyl-3-hydroxy-1-cyclohexenyl)-3,7-dimethyl-2,4,6,8-nonatetraenoic acid or a pharmaceutically usable salt thereof.

**Revendications**

1. Acide 9-(2,6,6-triméthyl-3-hydroxy-1-cyclohexényl)-3,7-diméthyl-2,4,6,8-nonatétraénoïque ou un de ses sels pharmaceutiquement acceptables.

2. Composition pharmaceutique contenant le composé acide 9-(2,6,6-triméthyl-3-hydroxy-1-cyclohexényl)-3,7-diméthyl-2,4,6,8-nonatétraénoïque ou un de ses sels pharmaceutiquement acceptables.